# EUROPEAN PATENT APPLICATION

(11) **EP 3 838 517 A1**
(43) Date of publication of application: **23.06.2021**
(21) Application number: 19849797.6
(22) Date of filing: 02.08.2019
(51) Int. Cl.: B25J 17/00, A61B 17/29, A61B 34/30

(54) **INSTRUMENT FOR SURGICAL ASSISTANCE ROBOT**

(30) Priority: 14.08.2018 JP 2018152642; 04.02.2019 JP 2019017778
(71) Applicant: NHK Spring Co., Ltd., Yokohama-shi, Kanagawa 236-0004 (JP); Riverfield Inc., Shinjuku-ku Tokyo 160-0017 (JP)
(72) Inventor: HIRATA, Takafumi, Yokohama-shi Kanagawa 236-0004 (JP); KUROKAWA, Shimpei, Yokohama-shi Kanagawa 236-0004 (JP); HOTODA, Yuki, Yokohama-shi Kanagawa 236-0004 (JP); HARAGUCHI, Daisuke, Tokyo 160-0017 (JP); SHINDO, Koki, Tokyo 160-0017 (JP); TAKIKAWA, Kyohei, Tokyo 160-0017 (JP)
(74) Representative: Oppermann, Frank
(86) International application number: PCT/JP2019/030573
(87) International publication number: WO 2020/036081

(57) **Abstract**

Provided is an instrument for a surgical assistance robot, capable of ensuring sufficient flexibility and rigidity in an axial direction. The instrument 100 for a surgical assistance robot is provided with a joint function part 109 at an axial front end portion of a shaft 103 being a rod-shaped member extended from a main body 101, the joint function part configured to bend with respect to the axial direction of the shaft 103, thereby to enable an orientation of the end effector 105 to be changed, wherein a bending structure 107 that enables the joint function part 109 to be bent is provided with an outer coiled part 5 formed of a wire 5a wound in a coiled shape and an inner coiled part 7 formed of a wire 7a wound in a coiled shape and arranged in the outer coiled part 5, wherein the outer coiled part 5 has a plurality of gaps 5c to distance adjacent coils 5b, and coils 7b of the inner coiled part 7 are provided so as to correspond to the gaps 5c of the outer coiled part 5 and fit between the adjacent coils 5b while being in contact with the adjacent coils 5b of the outer coiled part 5.

## Description

### FIELD OF THE INVENTION

The present invention relates to an instrument for a surgical assistance robot.

### BACKGROUND OF THE INVENTION

Robots, manipulators, actuators or the like in various fields may have joint function parts. As a bending structure applied to such a joint function part, there is a flexible member disclosed in Patent document 1, for example.

The flexible member of Patent document 1 is configured by swingably engaging a plurality of disc elements with each other to perform bending operation as a whole according to swinging of each disc element.

The flexible member having the structure smoothly performs the bending operation and assures rigidity against compression in an axial direction, to stabilize the bending operation.

The flexible member of Patent document 1, however, has a problem that the structure is complicated because the plurality of disc elements are engaged with each other.

### PRIOR ART DOCUMENT

### PATENT DOCUMENT

PATENT DOCUMENT 1: JP 2009-538186 A

### SUMMARY OF THE INVENTION

### PROBLEMS TO BE SOLVED BY THE INVENTION

A problem to be solved is that a structure is complicated if bending operation is stabilized.

### MEANS FOR SOLVING THE PROBLEM

The present invention mainly characterizes an instrument for a surgical assistance robot by a main body, a shaft being a rod-shaped member extended from the main body, an end effector for surgical operation provided at a front end of the shaft in an axial direction, and a joint function part provided at the front end to bend with respect to the axial direction of the shaft, thereby to enable an orientation of the end effector to be changed, wherein the joint function part has a bending structure that enables the bending, and the bending structure comprises an outer coiled part formed of a wire which is wound in a coiled shape to have a plurality of coils in the axial direction, and an inner coiled part formed of a wire which is wound in a coiled shape to have a plurality of coils in the axial direction and arranged in the outer coiled part, wherein the outer coiled part has a plurality of gaps to distance adjacent coils in the axial direction, and the coils of the inner coiled part are provided so as to correspond to the gaps of the outer coiled part and fit between the adjacent coils while being in contact with the adjacent coils of the outer coiled part.

### EFFECT OF THE INVENTION

According to the present invention, since the bending structure is configured by arranging the inner coiled part in the outer coiled part, the structure is simplified.

Further, since the coils of the inner coiled part fit between the adjacent coils while being in contact with the adjacent coils of the outer coiled part, rigidity in the axial direction is ensured.

Furthermore, at the time of the bending, the gaps of the outer coiled part are reduced on an inner side of the bending to displace the inner coiled part toward an outer side of the bending, and the gaps of the outer coiled part are enlarged on an outer side of the bending to allow the displacement of the inner coiled part, thereby to ensure sufficient flexibility even while the rigidity in the axial direction is ensured.

As a result, the present invention enables the structure to be simplified while stabilizing the bending operation.

Moreover, in the present invention, since the gaps of the outer coiled part are reduced on the inner side of the bending and the gaps of the outer coiled part are enlarged on the outer side of the bending, the length on the axis of the outer coiled part is not changed by comparison with a straight state. If it is used to guide a flexible member movably in the axial direction on an inner peripheral side, a moving amount of the flexible member is certainly kept constant.

### BRIEF DESCRIPTION OF THE DRAWINGS

[FIG. 1] It is a sectional view illustrating a bending structure (Reference example 1).
[FIG. 2] It is an enlarged view illustrating a part of the bending structure of IFG. 1 (Reference example 1).
[FIG. 3] It is a sectional view illustrating a bending state of the bending structure of IFG. 1 (Reference example 1).
[FIG. 4] It is an enlarged view illustrating a part of the bending structure of FIG. 3 (Reference example 1).
[FIG. 5] They are schematic sectional views illustrating a drop-off of an inner coiled part from an outer coiled part in which (A) is a state before the drop-off and (B) is a state after the drop-off (Reference example 1).
[FIG. 6] (A) is a sectional view illustrating a bending structure according to a comparative example, and (B) is a sectional view illustrating a bending state of the bending structure according to the comparative example (Reference example 1).
[FIG. 7] It is an enlarged sectional view illustrating a part of a bending structure (Reference example 2).
[FIG. 8] It is an enlarged sectional view illustrating a part of a bending structure (Reference example 3).
[FIG. 9] It is a sectional view illustrating a bending structure (Reference example 4).
[FIG. 10] It is an enlarged sectional view illustrating a part of a bending structure (Reference example 5).
[FIG. 11] It is a perspective view illustrating a part of robot forceps (Reference example 6).
[FIG. 12] It is a sectional view of the robot forceps of FIG. 11 (Reference example 6).
[FIG. 13] It is a perspective view illustrating a bendable part of the robot forceps of FIG. 11 (Reference example 6).
[FIG. 14] It is a sectional view of the bendable part of FIG. 13 (Reference example 6).
[FIG. 15] It is a schematic perspective view illustrating an instrument for a surgical assistance robot to which a bending structure is applied (Embodiment 1).
[FIG. 16] It is a perspective view illustrating an internal structure of a main body of the instrument for a surgical assistance robot of FIG. 16 is applied (Embodiment 1).
[FIG. 17] It is a plan view illustrating an internal structure of a main body of the instrument for a surgical assistance robot of FIG. 16 (Embodiment 1).
[FIG. 18] It is a plan view illustrating an internal structure of a main body of an instrument for a surgical assistance robot (Embodiment 2).
[FIG. 19] It is a partial perspective view explaining the internal structure of the main body of the instrument for a surgical assistance robot of FIG. 18 (Embodiment 2).

### EMBODIMENT FOR CARRYING OUT THE INVENTION

The object that a structure is simplified while bending operation is stabilized is accomplished by a bending structure having a double coil shape in which an inner coiled part is arranged in an outer coiled part.

An instrument for a surgical assistance robot, may comprise a main body, a shaft being a rod-shaped member extended from the main body, an end effector for surgical operation provided at a front end of the shaft in an axial direction, and a joint function part provided at the front end to bend with respect to the axial direction of the shaft, thereby to enable an orientation of the end effector to be changed. The joint function part has a bending structure that enables the bending, and the bending structure comprises an outer coiled part formed of a wire which is wound in a coiled shape to have a plurality of coils in the axial direction, and an inner coiled part formed of a wire which is wound in a coiled shape to have a plurality of coils in the axial direction and arranged in the outer coiled part, wherein the outer coiled part has a plurality of gaps to distance adjacent coils in the axial direction, and the coils of the inner coiled part are provided so as to correspond to the gaps of the outer coiled part and fit between the adjacent coils while being in contact with the adjacent coils of the outer coiled part.

### REFERENCE EXAMPLE 1

### [Structure of bending structure]

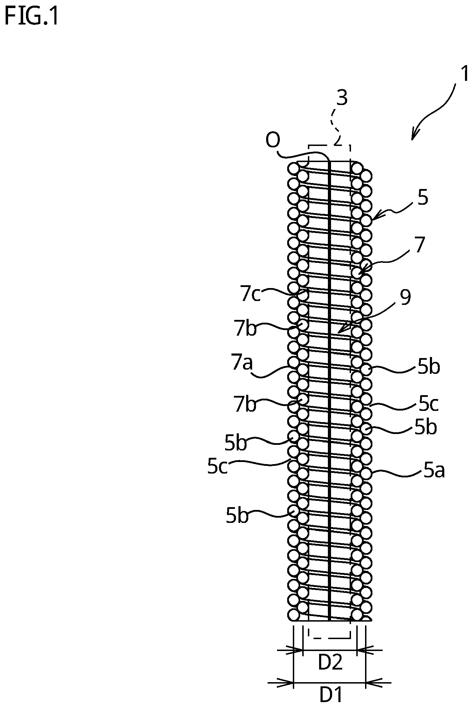

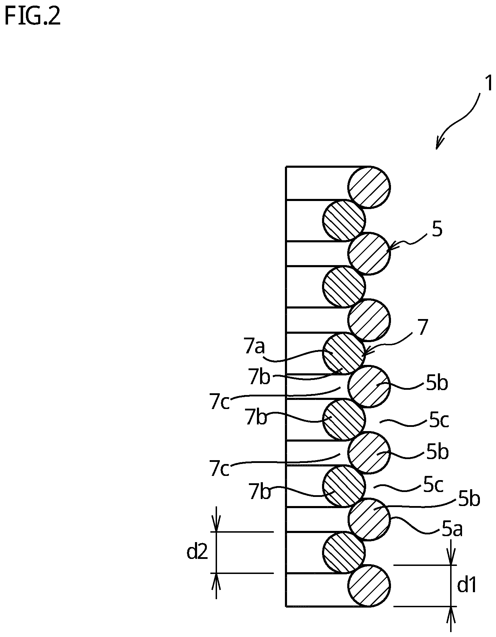

FIG. 1 is a sectional view illustrating a bending structure for a flexible member according to the reference example 1 of the present invention, and FIG. 2 is an enlarged view partly illustrating the same.

A bending structure 1 is one applied to a joint function part for, for example, robots, manipulators, actuators and the like in various fields. The bending structure 1 is provided between a base part and a movable part of the joint function part, and supports the movable part displaceably with respect to the base according to bending.

The bending structure 1 of the present reference example has a double coil shape, and is provided with an outer coiled part 5, and an inner coiled part 7. With the double coil shape, the bending structure 1 of the present reference example is bendable relatively to an axial direction, has a length of a central axis or axis O in the axial direction is approximately constant before, after and during bending based on that an inner diametral side of the bending is compressed and an outer diametral side of the bending is extended when the bending is performed according to external force, and restricts compression in the axial direction when the bending is not performed or the like. The bending structure 1 of the present reference example is also provided with a flexible member 3 serving as a restricting member.

The flexible member 3 passes through the bending structure 1 being movably in the axial direction, and restricts deviation of the inner and the outer coiled parts 5, 7 in a diametral direction although the details will be explained later. The flexible member 3 of the present reference example is configured by using, for example, a push-pull cable or the like. According to this, the bending structure 1 has a function to guide the flexible member 3 in the axial direction, and is bendable as well as the flexible member 3 according to bending operation of the joint function part.

It should be noted that the bending means that the axis O of the joint function part or the bending structure 1 is curved or bent. Further, the flexible member 3 may be omitted.

The outer coiled part 5 is a coil spring, and is made of a wire 5a would in a coiled shape. The outer coiled part 5, therefore, has a plurality of coils 5b in the axial direction. It should be noted that the coil 5b means one turn for composing the coiled shape (the same applies to the following).

The material of the wire 5a may be metal, resin or the like. The sectional shape of the wire 5a is formed into a circular shape, but may be oval or the like.

A mean diameter D1 of the outer coiled part 5 is constant from one end to the other end in the axial direction. The mean diameter D1 of the outer coiled part 5 may be, however, varied in the axial direction.

The outer coiled part 5 has a plurality of gaps 5c to axially distance adjacent coils 5b in the axial direction. The gaps 5c of the present reference example are formed in respective interspaces of the adjacent coils 5c in the axial direction, and all the gaps 5c have the same dimension in the axial direction. The gaps 5c may be, however, provided in only some interspaces between the coils 5c in the axial direction. Further, the dimensions of the gaps 5c in the axial direction may be varied.

The inner coiled part 7 is a coil spring, and is made of a wire 7a would in a coiled shape having a plurality of coils 7b in the axial direction. In the inner coiled part 7, in the same way as the outer coiled part, the material of the wire 7a may be metal, resin or the like, and the sectional shape of the wire 7a is formed into a circular shape, but may be oval or the like.

The inner coiled part 7 is arranged inside the outer coiled part 5, and defines a passing portion 9 through which the flexible member 3 is passed on the inner circumference. The inner coiled part 7 of the present reference example is screwed into the outer coiled part 5. With this screwing, the coils 7b of the inner coiled part 7 are arranged in the respective interspaces of the adjacent coils 5b of the outer coiled part 5. The inner coiled part 7 has, therefore, a structure in which the coils 7b are provided so as to correspond to the gaps 5c of the outer coiled part 5.

Further, the coils 7b of the inner coiled part 7 fit between the adjacent coils 5b while being in contact with the adjacent coils 5b of the outer coiled part 5 according to settings of a mean diameter D2 and a wire diameter d2 of the wire 7a.

In addition, the mean diameter D2 of the inner coiled part 7 is constant from one end to the other end in the axial direction. The mean diameter D2 of the inner coiled part 7 may be, however, varied in the axial direction according to the mean diameter D1 of the outer coiled part 5 or the like.

Further, the wire diameter d2 of the wire 7a is the same as the wire diameter d1 of the wire 5a of the outer coiled part 5. The wire diameter d2 of the wire 7a may be, however, greater or less than the wire diameter d1 of the wire 5a of the outer coiled part 5.

The inner coiled part 7 has a plurality of gaps 7c to distance adjacent coils 7b in the axial direction. The gaps 7c are formed in respective interspaces of the adjacent coils 7c in the axial direction according to the screwing into the outer coiled part 5, and all the gaps 7c have the same dimension in the axial direction.

It should be noted that the outer coiled part 5 and the inner coiled part 7 may have, other than the structure having the gaps 5c, 7c in the respective interspaces between the adjacent coils 5b, 7b in a free state in which the inner coiled part 7 is not arranged in the outer coiled part 5, a structure (close contact spring) in which the adjacent coils 5b, 7b are closely contact with each other in the free state. Further, only one of the outer coiled part 5 and the inner coiled part 7 may be the close contact spring.

In a case that the outer coiled part 5 and the inner coiled part 7 are the close contact springs in the free state, the inner coiled part 7 and the outer coiled part 5 are screwed together, thereby to mutually distance the interspaces between the coils 5b, 7b and form the gaps 5c of the outer coiled part 5 and the gaps 7c of the inner coiled part 7. In this case, initial tension may be applied to the bending structure 1 having the double coil shape.

### [Operation of the bending structure]

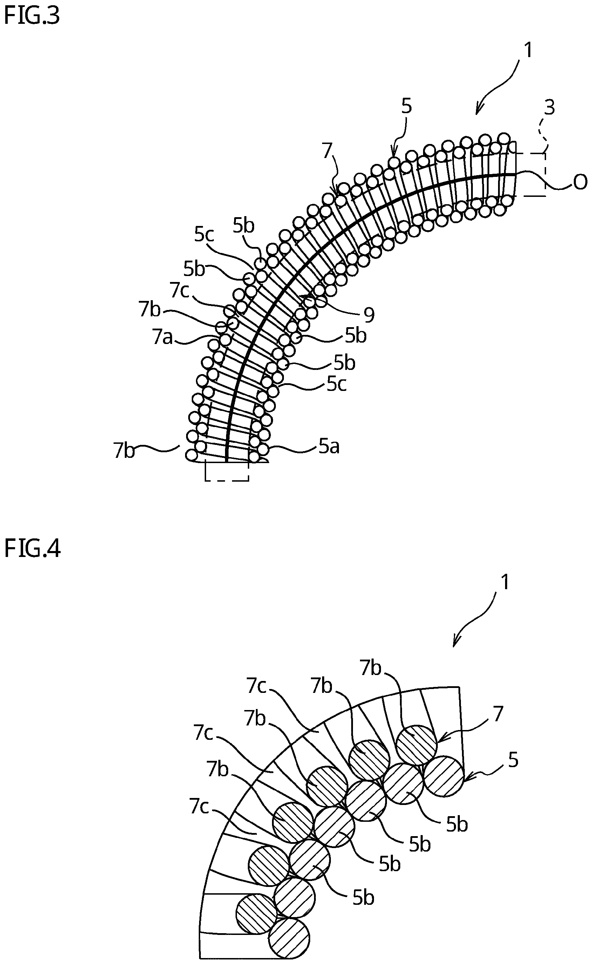

FIG. 3 is a sectional view illustrating a bending state of the bending structure of FIG. 1, and FIG. 4 is an enlarged view partly illustrating the same.

In the bending structure 1, as illustrated in FIGs. 1 and 2, the coils 7b of the inner coiled part 7 fit between the adjacent coils 5b while being in contact with the adjacent coils 5b of the outer coiled part 5 when the axis O (being also the axis of the outer coiled part 5) is in a straight state without bending.

Accordingly, the bending structure 1 prevents, by the coils 7b of the inner coiled part 7, the gaps 5c of the outer coiled part 5 from being compressed to be prevented from being compressed as a whole even if compressive force acts in the axial direction. It should be noted that the gaps 7c of the inner coiled part 7 are prevented by the coils 5b of the outer coiled part 5 from being compressed if the inner coiled part 7 is as a basis.

The bending structure 1, therefore, prevents compression of itself and the joint function part to which the bending structure is applied. As a result, when guiding movement of the flexible member 3 in the axial direction, the length of the axis O and a moving amount of the flexible member 3 passing on the axis O are kept constant to also ensure stability of operation of the flexible tube 3.

As illustrated in FIGs. 3 and 4, when the axis O of the bending structure 1 is bent, the gaps 5c of the outer coiled part 5 are reduced on the inner side of the bending and the gaps 5c of the outer coiled part 5 are enlarged on the outer side of the bending.

At this time, the bending structure 1 smoothly bends by the inner coiled part 7 displacing outwardly in the diametral direction.

Namely, each coil 7b of the inner coiled part 7 is pushed inwardly in the diametral direction by reduction of the gaps 5c of the outer coiled part 5 on the inner side of the bending of the bending structure 1. According to this, the inner coiled part 7 as a whole is displaced outwardly in the diametral direction, and this displacement is allowed so that each coil 7b of the inner coiled part 7 enters into the enlarged gap 5c of the outer coiled part 5.

The bending structure 1, therefore, is the structure in which the compression is prevented in the axial direction whereas the flexibility is never hindered. As a result, the bending structure 1 is stabilized in the bending operation.

Further, when the bending structure 1 is bent, the gaps 5c of the outer coiled part 5 are reduced on the inner side of the bending and the gaps of the outer coiled part 5 are enlarged on the outer side of the bending as mentioned above. The size of the gaps 5c on the axis O is not changed by comparison with the straight state.

The bending structure 1, therefore, keeps the length of the axis O and the moving amount of the flexible member 3 passing on the axis O of the bending structure 1 constant at the time of not only the straight state, but the bending, thereby to ensure the stability of the operation of the flexible member 3.

Further, the bending structure 1 of the present reference example brings the adjacent coils 5b of the outer coiled part 5 into contact with each other on the inner side of the bending when bending at the predetermined angle (FIG. 4).

In the bending structure 1, therefore, the length on the axis O starts to increase from when the coils 5b are brought into contact with each other. Accordingly, the bending at the predetermined angle or more is notified to an operator of the joint function part according to the change in the moving amount of the flexible member 3.

At the time of the bending operation of the bending structure 1, the inner coiled part 7 is prevented from being dropped off from the outer coiled part 5 by the flexible member 3.

Namely, the inner coiled part 7 as a whole is displaced outwardly in the diametral direction so that each coil 7b of the inner coiled part 7 enters into the enlarged gap 5c of the outer coiled part 5 at the time of the bending of the bending structure 1.

This displacement (movable length in which the inner coiled part 7 is movable in the diametral direction relatively to the axis O of the outer coiled part 5) is equal to or less than half of (the diameter of the outer coiled part - the diameter of the inner coiled part). In addition, the diameters mean the mean diameters D1 and D2 of the outer coiled part 7 and the inner coiled part 5. The diameters may be, however, outer diameters or inner diameters of the outer coiled part 7 and the inner coiled part 5.

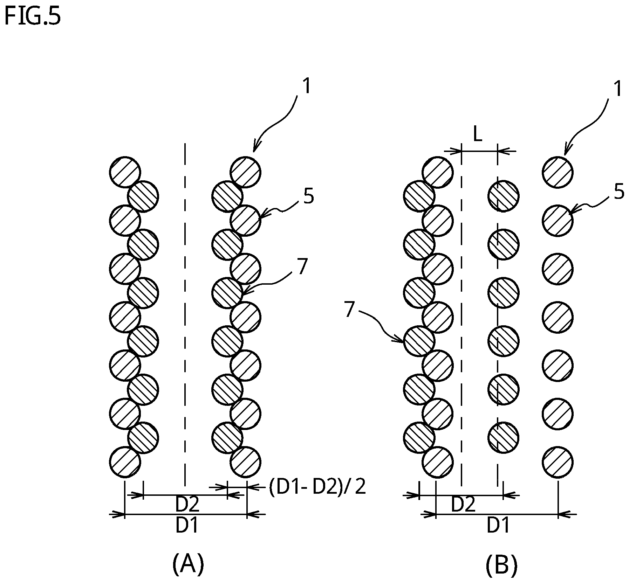

FIGs. 5 are schematic sectional views illustrating a drop-off of the inner coiled part 7 from the outer coiled part 5 in which (A) is a state before the drop-off and (B) is a state after the drop-off.

As illustrated in FIGs. 5, if a moving amount L in the diametral direction of the inner coiled part 7 in the straight state exceeds (D1-D2)/2 being half of (the diameter of the outer coiled part 5 - the diameter of the inner coiled part 7), the inner coiled part 7 gets over the outer coiled part 5 to be in a drop-off state. In addition, in FIG. 5, the moving amount L is indicated as a deviation amount between the axis of the inner coiled part 7 and the axis of the outer coiled part 5.

At the time of the bending of the bending structure 1, if the moving amount L of the inner coiled part 7 in the diametral direction exceeds (D1-D2)/2 being a half of (the diameter of the outer coiled part 5 - the diameter of the inner coiled part 7), it results in causing the drop-off as illustrated in FIG. 5 when returning to the straight state. In the present reference example, therefore, the movable length in which the inner coiled part 7 is movable in the diametral direction relatively to the axis O of the outer coiled part 5 is equal to or less than (D1-D2)/2 being half of (the diameter of the outer coiled part 5 - the diameter of the inner coiled part 7).

The movable length is set by passing the flexible member 3 through the bending structure 1 in the present reference example. In this way, in the present reference example, the drop-off of the inner coiled part 7 from the outer coiled part 5 is prevented by the flexible member 3. The movable length may be, however, set by the setting of any one or both of the wire diameters d1 and d2 of the outer coiled part 5 and the inner coiled part 7 in a case that the bending structure 1 does not passes the flexible member 3 therethrough or a case that the diameter of the flexible member 3 is thin in a degree by which the aforementioned movable length cannot be set.

### [Moving amount of the comparative example]

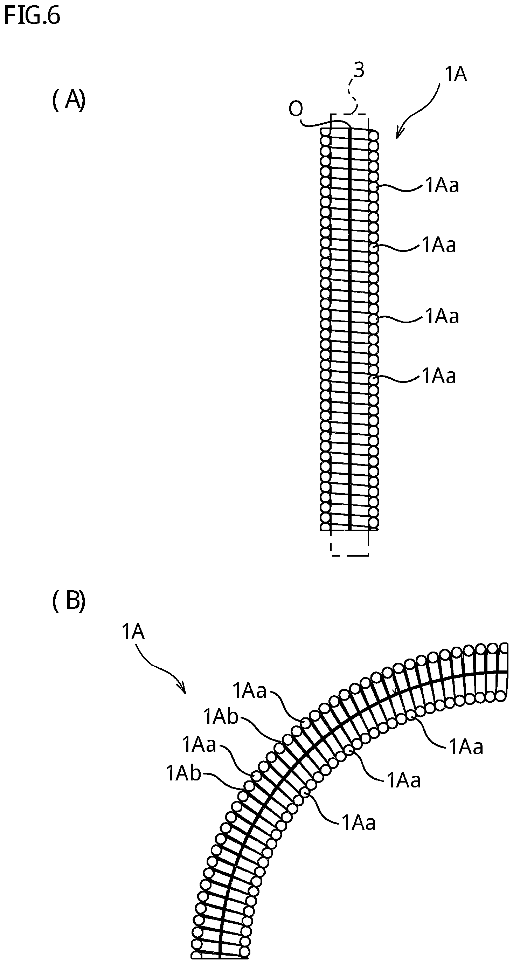

FIG. 6(A) is a sectional view illustrating a bending structure according to a comparative example, and FIG. 6(B) is a sectional view illustrating a bending state of the same.

The bending structure 1A according to the comparative example comprises of only a close contact spring, and is configured to be bendable and prevented from being compressed.

The bending structure 1A, at the time of bending, keeps a state in which coils 1Aa are in contact with each other on an inner side of the bending and forms gaps between the coils 1Aa on an outer side of the bending.

As this result, gaps 1Ab are also formed on a central portion of bending inner side and outer side of the bending structure 1 at the time of the bending. By the gaps 1Ab of the bending structure 1, a length of the axis O and the moving amount of the flexible member 3 passing on the axis O are increased.

Accordingly, the comparative example is one which does not ensure stability of operation of the flexible member 3 when guiding the flexible member 3 unlike the reference example 1.

### [Effect of the reference example 1]

As mentioned above, the bending structure 1 of the present reference example is the bending structure which movably passes the flexible member 3 therethrough in the axial direction and is bendable as well as the flexible member 3. The bending structure is provided with the outer coiled part 5 formed of the wire 5a which is wound in the coiled shape to have the plurality of coils 5b in the axial direction, and the inner coiled part 7 formed of the wire 7a which is wound in the coiled shape to have the plurality of coils 7b in the axial direction and arranged in the outer coiled part 5.

The outer coiled part 5 has the plurality of the gaps 5c to distance the adjacent coils 5b, and the coils 7b of the inner coiled part 7 are provided so as to correspond to the gaps 5c of the outer coiled part 5 and fit between the adjacent coils 5b while being in contact with the adjacent coils 5b of the outer coiled part 5.

The bending structure 1, therefore, is formed by arranging the inner coiled part 7 into the outer coiled par 5, so that the structure is simplified.

Further, the bending structure 1 prevents, by the coils 7b of the inner coiled part 7, the gaps 5c of the outer coiled part 5 from being compressed to be prevented from being compressed as whole even if the compressive force acts in the axial direction. Accordingly, the bending structure 1 ensures the rigidity in the axial direction in a degree not to cause the joint function part to be compressed.

Further, the inner coiled part 7 is displaced toward the outer side of the bending while the gaps 5c of the outer coiled part 5 are reduced on the inner side of the bending at the time of the bending, and the gaps of the outer coiled part 5 are enlarged on the outer side of the bending to allow the displacement of the inner coiled part 7, thereby to ensure sufficient flexibility to bend as well as the joint function part even while the rigidity in the axial direction is ensured.

As a result, the bending structure 1 enables its structure to be simplified while stabilizing the bending operation, so that it ensures stability of operation of a device such as a robot, manipulator, or actuator having the joint function part.

Further, in the bending structure 1 of the present reference example, the gaps 5c of the outer coiled part 5 are reduced on the inner side of the bending and the gaps 5c of the outer coiled part 5 are enlarged on the outer side of the bending. The length of the axis O of the outer coiled part 5 is not changed by comparison with the straight state, and the moving amount of the flexible member 3 is certainly kept constant.

Accordingly, stability of the operation of the flexible member 3 is ensured and therefore the stability of operation of the device having the joint function part is further ensured.

Further, in the present reference example, the movable length (displacement amount) in which the inner coiled part 7 is movable in the diametral direction relatively to the axis O of the outer coiled part 5 is equal to or less than half of (the diameter of the outer coiled part - the diameter of the inner coiled part). The inner coiled part 7, therefore, is prevented from being dropped off from the outer coiled part 5.

Furthermore, in the present reference example, the flexible member 3 as the restricting member restricts the movement of the inner coiled part 7 so that the movable length is equal to or less than half of (the diameter of the outer coiled part - the diameter of the inner coiled part). The inner coiled part 7 is, therefore, easily prevented from being dropped off without changing shapes of the inner coiled part 7 and the outer coiled part 5.

The bending structure 1 movably passes the flexible member 3 therethrough in the axial direction and is bendable together with the flexible member 3. The inner coiled part 7 is, therefore, prevented from being dropped off by using the flexible member 3 in the aspect to guide the flexible member 3.

In the present reference example, the outer coiled part 5 has the gaps 5c in the respective interspaces between the adjacent coils 5b in the axial direction, so that the bending structure 1 is bent smoothly.

In the present reference example, the inner coiled part 7 and the outer coiled part 5 are formed separately and the inner coiled part 7 is screwed into the outer coiled part 5, so that the assembly is easily performed. Further, characteristics of the bending structure 1 are easily varied by varying characteristics of any one or both of the inner coiled part 7 and the outer coiled part 5.

Further, since the bending structure 1 of the present reference example brings the adjacent coils 5b of the outer coiled part 5 into contact with each other on the inner side of the bending when bending at the predetermined angle, the bending at the predetermined angle or more is notified to an operator of the joint function part according to the change in the moving amount of the flexible member 3.

### REFERENCE EXAMPLE 2

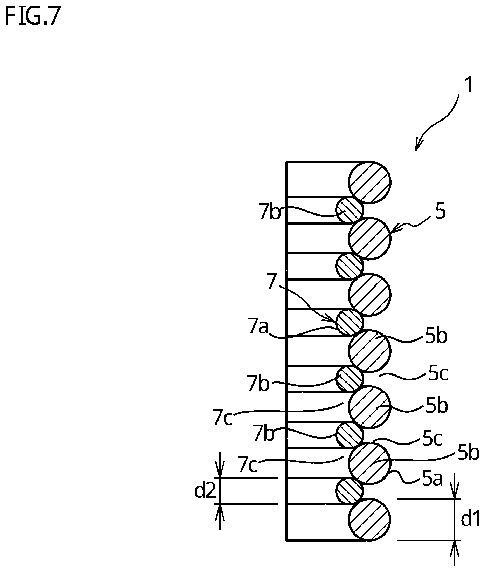

FIG. 7 is an enlarged sectional view illustrating a part of a bending structure according to the reference example 2. In addition, components in the reference example 2 corresponding to in the reference example 1 are represented with the same reference numerals to eliminate duplicate explanation.

A bending structure 1 of the reference example 2 is one in which a wire diameter d1 of a wire 5a of an outer coiled part 5 is different from a wire diameter d2 of a wire 7a of an inner coiled part 7. In the reference example 2, the wire diameter d1 of the outer coiled part 5 is set greater than the wire diameter d2 of the inner coiled part 7. It should be noted that the wire diameter d1 of the outer coiled part 5 may be smaller than the wire diameter d2 of the inner coiled part 7.

The bending structure 1 provides the same effect as the reference example 1 even in the case that the wire diameter d1 of the outer coiled part 5 is different from the wire diameter d2 of the inner coiled part 7 in this way. Further, a free length and characteristics of the bending structure 1 are adjusted by differing the wire diameter d2 and the wire diameter d1 from each other.

### REFERENCE EXAMPLE 3

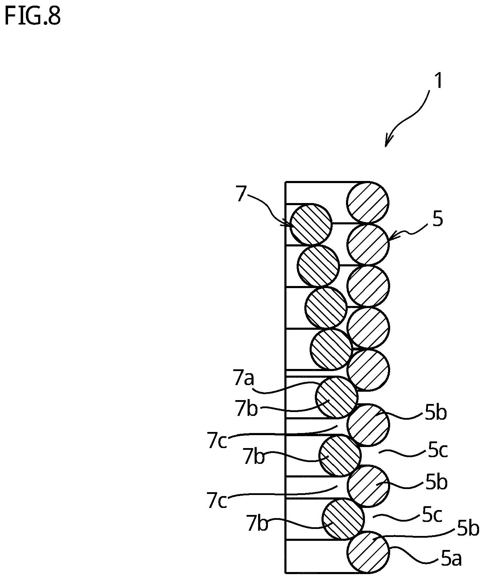

FIG. 8 is an enlarged sectional view illustrating a part of a bending structure according to the reference example 3. In addition, components in the reference example 3 corresponding to in the reference example 1 are represented with the same reference numerals to eliminate duplicate explanation.

A bending structure 1 of the reference example 3 fits coils 7b of an inner coiled part 7 between adjacent coils 5b while be in contact with the adjacent coils 5b of an outer coiled part 5 in part of the outer coiled part 5 in an axial direction.

Namely, the inner coiled part 7 is formed so that a mean diameter D2 (see FIG. 1) becomes gradually small in the axial direction. According to this, the inner coiled part 7 fits between the adjacent coils 5b of the outer coiled part 5 only in the part of the outer coiled part 5 in the axial direction as mentioned above.

In addition, in the present reference example, the inner coiled part 7 and the outer coiled part 5 are respective close contact springs and gaps 5c of the outer coiled part 5 are reduced as reduction of the mean diameter D2 of the inner coiled part 7.

Even this structure provides the same effect as the reference example 1. Additionally, the present reference example fits the coils 7b of the inner coiled part 7 between the adjacent coils 5b of the outer coiled part 5 only in the part of the outer coiled part 5 in the axil direction, thereby to adjust a free length and characteristics of the bending structure 1.

### REFERENCE EXAMPLE 4

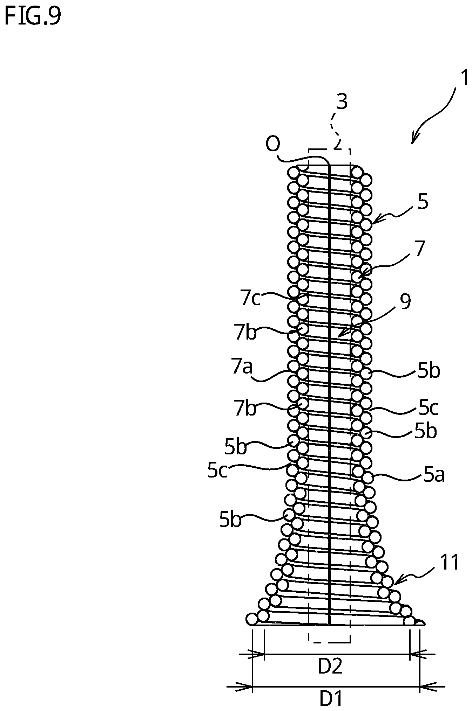

FIG. 9 is a sectional view illustrating a bending structure according to the reference example 4. In addition, components in the reference example 4 corresponding to in the reference example 1 are represented with the same reference numerals to eliminate duplicate explanation.

A bending structure 1 of the reference example 4 is provided with an enlarged diameter portion 11 gradually increasing in diameter in part in an axial direction. In the present reference example, the enlarged diameter portion 11 is provided at one end of the bending structure 1 in the axial direction. The enlarged diameter portion 11 may be provided in the middle of or at the other end of the bending structure 1 in the axial direction.

At the enlarged diameter portion 11, both mean diameters D1 and D2 of an outer coiled part 5 and an inner coiled part 7 are enlarged gradually, and it keeps a state in which coils 7b of the inner coiled part 7 fit between coils 5b while be in contact with the adjacent coils 5b of the outer coiled part 5.

Even this structure provides the same effect as the reference example 1. Further, the enlarged diameter portion 11 adjusts characteristics of the bending structure 1.

### REFERENCE EXAMPLE 5

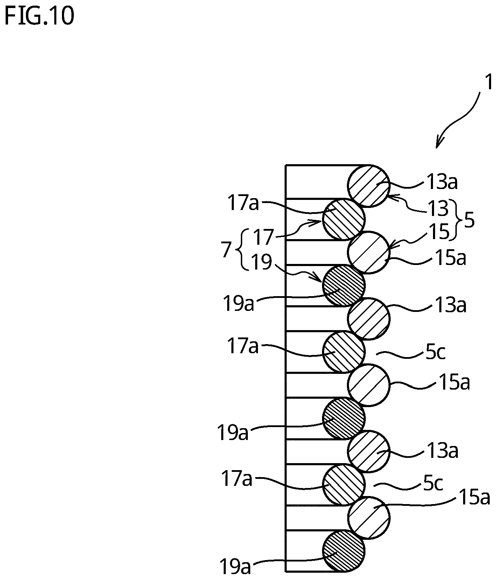

FIG. 10 is an enlarged sectional view illustrating a part of a bending structure according to the reference example 5. In addition, components in the reference example 5 corresponding to in the reference example 1 are represented with the same reference numerals to eliminate duplicate explanation.

A bending structure 1 of the reference example 5 is one in which an outer coiled part 5 and an inner coiled part 7 are respectively configured by two coil parts. In particular, the outer coiled part 5 is composed of a first outer coiled part 13 and a second outer coiled part 15 and the inner coiled part 7 is composed of a first inner coiled part 17 and a second inner coiled part 19.

In the first outer coiled part 13 and the second outer coiled part 15, coils 13a, 15a are alternately arranged in an axial direction, and in also the first inner coiled part 17 and the second inner coiled part 19, coils 17a, 19a are alternately arranged in the axial direction.

Namely, in the outer coiled part 5, the coils 13a, 15a of the first outer coiled part 13 and the second outer coiled part 15 are adjacent to each other in the axial direction to form gaps 5c between those adjacent coils 13a, 15a.

The coils 17a of the first inner coiled part 17 and the coils 19a of the second inner coiled part 19 of the inner coiled part 7 respectively fit between the coils 13a, 15a while being in contact with the coils 13a, 15a of the first outer coiled part 13 and the second outer coiled part 15.

Even this structure provides the same effect as the reference example 1 and adjusts a free length and characteristics of the bending structure 1.

It should be noted that the number of coiled parts composing the outer coiled part 5 and the inner coiled part 7 may be varied. Further, only one of the outer coiled part 5 and the inner coiled part 7 may be composed of a plurality of coiled parts.

### REFERENCE EXAMPLE 6

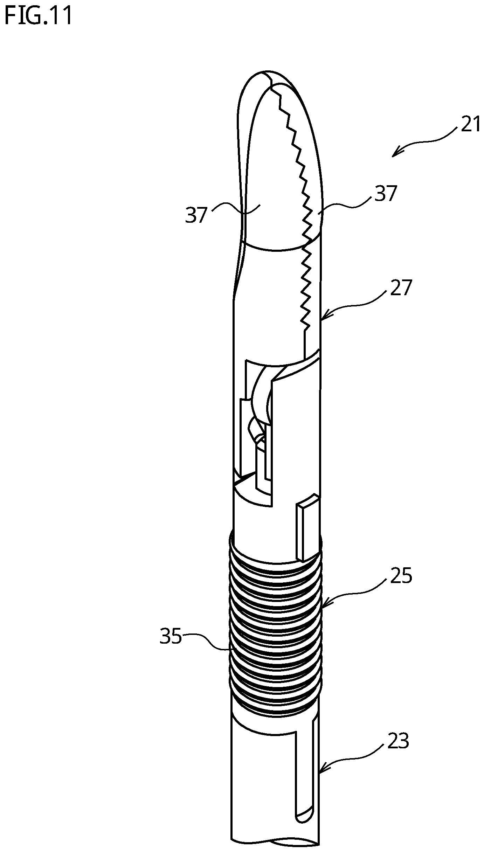

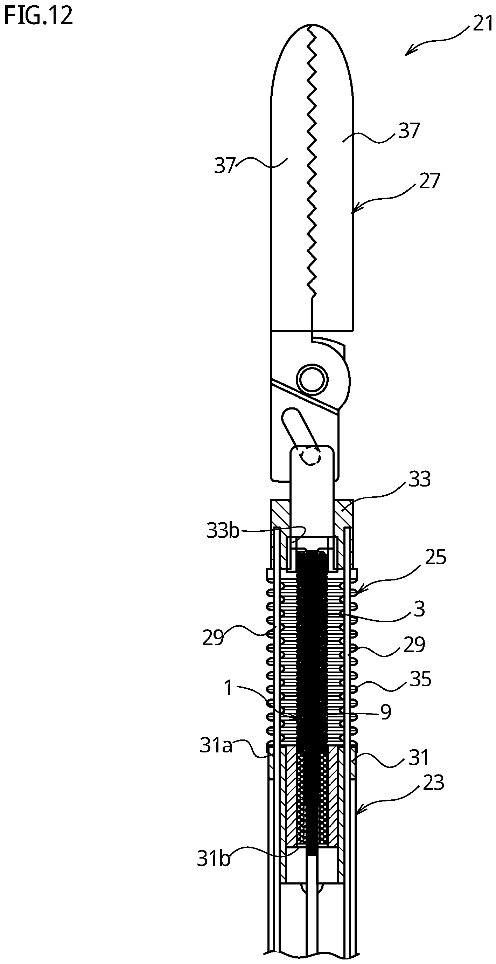

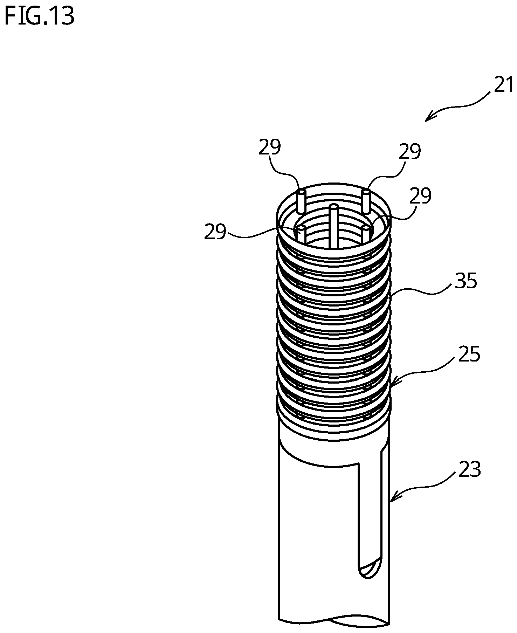

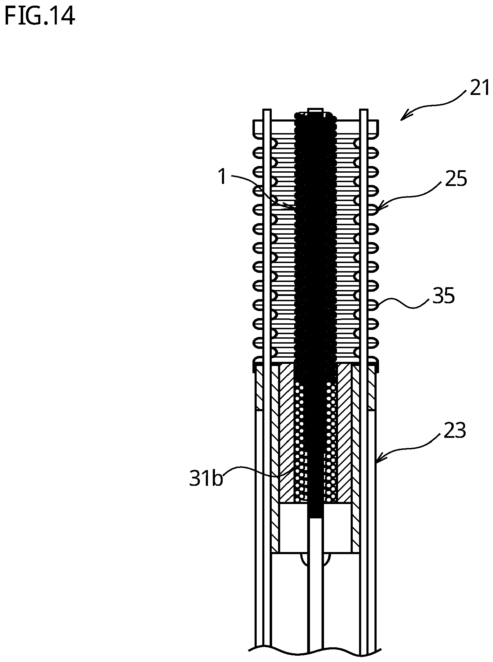

FIG. 11 is a perspective view illustrating a part of robot forceps to which a bending structure is applied according to the reference example 6 of the present invention, FIG. 12 is a sectional view of the same, FIG. 13 is a perspective view illustrating a joint function part of the robot forceps of FIG. 11, and FIG. 14 is a sectional view of the same. In addition, components in the reference example 6 corresponding to in the reference example 1 are represented with the same reference numerals to eliminate duplicate explanation.

Robot forceps 21 of the present reference example is one which forms a front end of a robot arm of a surgical robot as a medical manipulator.

In addition, the robot forceps 21 are an example of a device having a joint function part. The device having a joint function part is not limited to the medical manipulator as mentioned above. Namely, the device having the joint function part is not particularly limited, but a robot in the other field, every kind of manipulators, actuators or the like as long as it has the joint function part performing bending operation and moves a flexible member 3 in an axial direction to perform operation or the like. Further, in a case of a medical manipulator, an endoscope camera, manual forceps and the like that are not attached to surgical robots are included.

The robot forceps 21 of the present reference example is composed of a shaft 23, a joint function part 25, and a holding unit 27 as an end effector for surgical operation.

The shaft 23 is formed into, for example, a cylindrical shape. Passed in the shaft 23 are driving wires 29 to drive the joint function part 25 and a flexible member 3 being a push-pull cable to drive the holding unit 27. On a front end side of the shaft 23, the holding unit 27 is provided through the joint function part 25.

The joint function part 25 is provided with a base part 31, a movable part 33, a flexible tube 35, and a bending structure 1.

The base part 31 is a cylinder body formed of resin, metal or the like, and is attached at the front end of the shaft 23. Through the base part 31, the driving wires 29 pass via through-holes 31a in the axial direction and the flexible member 3 passes via an insertion hole 31b on an axial center portion.

The movable part 33 is a cylinder body formed of resin, metal or the like, and is attached to the holding unit 27 explained later. To the movable part 33, front end portions of the driving wires 29 are fixed. Accordingly, the movable part 33 is displaced with respect to the base part 31 by operation of the driving wires 29 to orient the holding unit 27 toward a desired direction. An axial center portion of the movable part 33 is provided with an insertion hole 33b to pass the flexible member 3 therethrough.

The flexible tube 35 is interposed between the base part 31 and the movable part 33 and is bent according to the displacement of the movable part 33 relative to the base part 31. The flexible tube 35 passes the driving wires 29 and the flexible member 3 therethrough in the axial direction.

The flexible tube 35 of the present reference example is configured by a bellows formed of a tube body having a wave sectional shape. The flexible tube 35 may use, however, a coil spring, a tube body or the like, and is not particularly limited as long as it has a flexible tube shape.

The bending structure 1 is the same as of the reference example 1. The bending structure 1 is arranged along the axial center portion of the flexible tube 35 and is provided between the base part 31 and the movable part 33. In addition, the bending structure 1 of any one of the reference examples 2-5 may be applied to the joint function part 25.

The bending structure 1 passes the flexible member 3 through the passing portion 9, in this state both ends of which are respectively attached to the insertion holes 31b and 33b of the base part 31 and the movable part 33. With this, the bending structure 1 supports the movable part 33 with respect to the base part 31 so as not to be movable in the axial direction and is bendable as well as the flexible member 3 according to the displacement of the movable part 33 relative to the base part 31.

The holding unit 27 has a pair of holding parts 37 openably pivotally supported with respect to the movable part 33 of the joint function part 25. The holding unit 27 is connected to the flexible member 3 passing through the joint function part 25 and is configured to open and close the holding parts 37 by axial movement (reciprocation movement) of the flexible member 3. It should be noted that the end effector is not limited to the holding unit 27 and may be, for example, scissors, a holding retractor, a needle driver or the like.

In the robot forceps 21 having the structure, an operator such as a doctor reciprocatingly operates the flexible member 3, thereby to cause the holding parts 37 of the holding unit 27 to perform opening/closing operation.

Further, the operator pulls any one or some of the driving wires 29 to bend the joint function part 25, thereby to cause the holding unit 27 to orient relatively to the shaft 23 toward a desired direction. In this state, if the flexible member 3 is reciprocated, the holding parts 37 of the holding unit 27 is caused to perform the opening/closing operation.

The opening/closing operation is stabilized and accurately performed because the moving amount of the flexible member 3 is constant as mentioned in the reference example 1.

Additionally, the present reference example provides the same effect as the reference example 1.

### EMBODIMENT 1

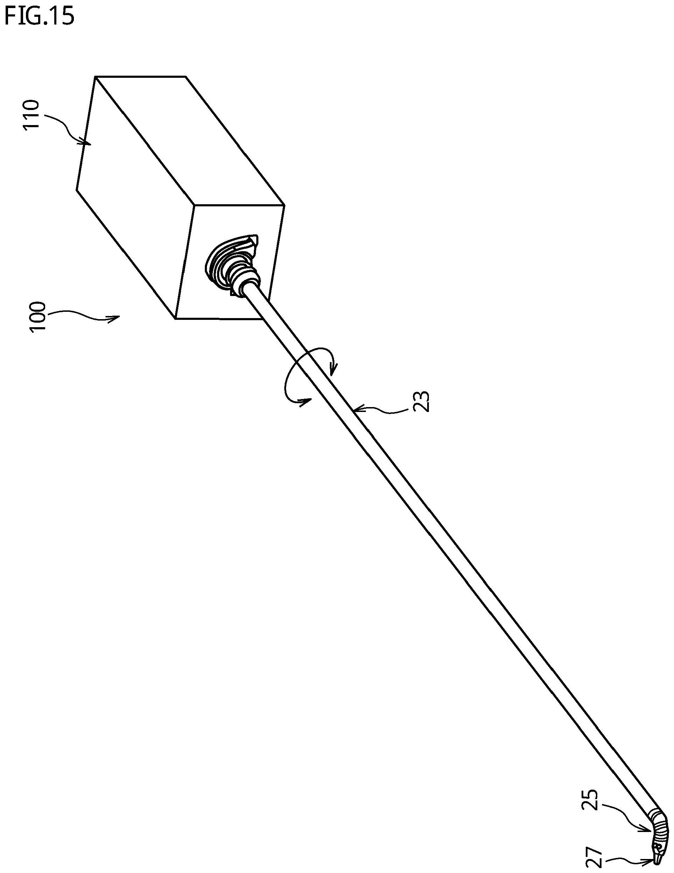

FIG. 15 is a schematic perspective view illustrating an instrument for a surgical assistance robot to which a bending structure is applied according to the embodiment 1 of the present invention. In addition, components in the embodiment 1 corresponding to in the reference examples 1 and 6 are represented with the same reference numerals to eliminate duplicate explanation.

The present embodiment is an instrument 100 for a surgical assistance robot to which the bending structure 1 of the reference example 1. The instrument 100 for a surgical assistance robot may, however, apply the bending structure 1 of any one of the reference examples 2-5. The instrument 100 for a surgical assistance robot is attached to a master-slave type surgical robot as one exchangeable and is driven by driving force from the surgical robot. In addition, the instrument 100 for a surgical assistance robot corresponds to one in which the robot forceps 21 of the reference example 6 is made as an exchangeable unit.

The instrument 100 for a surgical assistance robot, as illustrated in FIG. 15, is provided with a main body 110, a shaft 23, a holding unit 27 for surgical operation, and a joint function part 25.

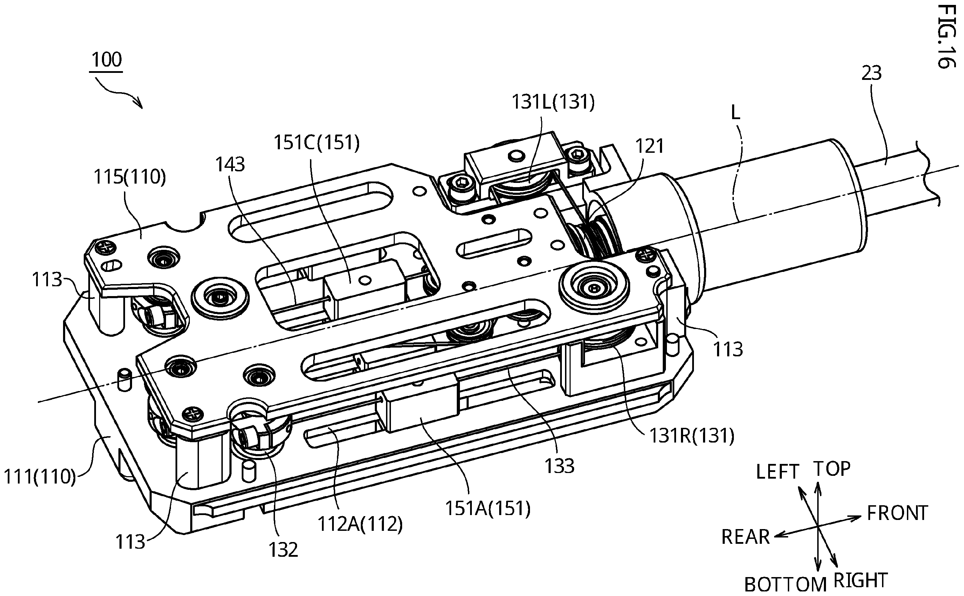

In addition, the present embodiment will provide explanation on the premise that a direction (axial direction) of an axis L in the shaft 23 is a back-and-forth direction, a direction which is orthogonal to the back-and-forth direction and in which a pair of main body side pulleys 131L, 131R are arranged side by side is a right-and-left direction, and a direction orthogonal to the back-and-forth direction and the right-and-left direction is a vertical direction to make the explanation simple (See FIG. 16).

The main body 110 forms a base part of the instrument 100 for a surgical assistance robot. To the main body 110, the shaft 23 being a rod member, in particular a cylindrical member, is extended along the back-and-forth direction.

At a front end portion of the shaft 23 in the back-and-forth direction (axial direction), the holding unit 27 is provided as an end effector for surgical operation. It should be noted that the end effector is not limited to the holding unit 27 and may be, for example, scissors, a holding retractor, a needle driver or the like similar to the reference example 6. Further, at the front end portion of the shaft 23, the joint function part 25 is provided. The joint function part 25 has the bending structure 1 and bends relatively to the back-and-forth direction to enable an orientation of the holding unit 27 to be changed.

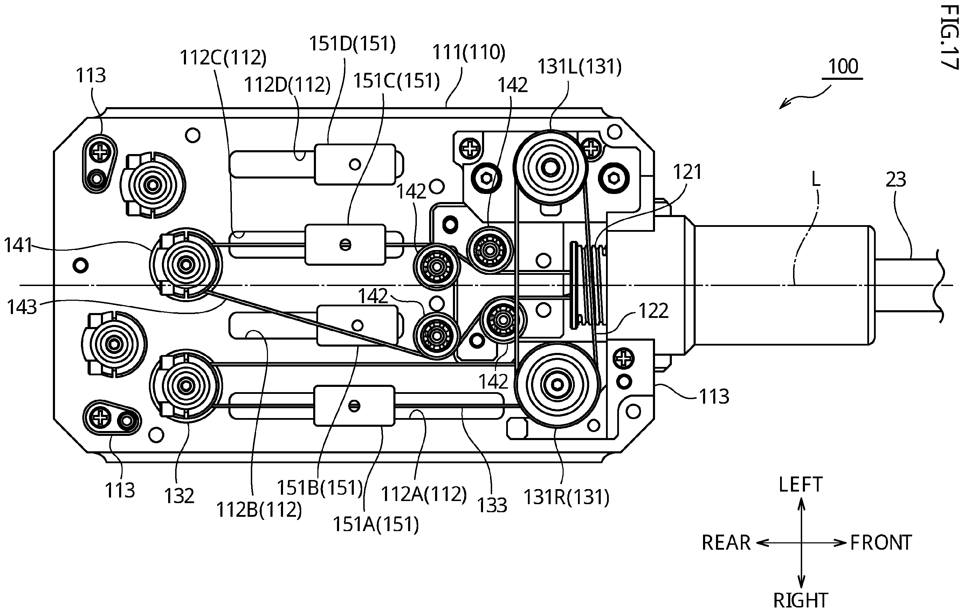

Hereinafter, an example of the main body 110 will explained in detail with reference to also FIGs. 16 and 17. It should be noted that the main body 110 is not limited to the configuration illustrated in FIGs. 16 and 17 and may employ the other configuration. Further, the shaft 23, the holding unit 27, and the joint function part 25 have the same configurations as of the refence example 6 and the details thereof will be omitted by reference of the reference example 6.

FIG. 16 is a perspective view illustrating an example of an internal structure of the main body 110 of the instrument 100 for a surgical assistance robot and FIG. 17 is a plan view of the same.

The main body 110 is formed into a rectangular parallelepiped shape inside which a shaft side pulley 121, the pair of main body side pulleys 131L, 131R, a drive pulley 132, a pulley 141 for forceps, guide pulleys 142, movable parts 151 and the like are stored.

In addition, in FIG. 15 only a base board 111 and a top board 115 of the main body 110 are illustrated and a member and the like covering a circumference are omitted to be illustrated in order to explain the shaft side pulley 121, the pair of the main body side pulleys 131L, 131R, the drive pulley 132, the pulley 141 for forceps, the guide pulleys 142, the movable parts 151 and the like arranged inside the main body 110. In FIG. 16, the top board 115 is further omitted to be illustrated.

The base board 111 of the main body 110 is one supporting the shaft 23 and the shaft side pulley 121 rotatably around the axis L at a center of a forward end side in a front direction. Further, it is what the main body side pulleys 131, the drive pulley 132, the pulley 141 for the forceps and the guide pulleys 142 are rotatably arranged on an upward face of the base board 111.

Further, to the base board 111, four slits 112 formed into a long hole shape extending in the back-and-forth direction are arranged side by side at intervals in the right-and-left direction. To the four slits 112, the single movable parts 151 are arranged movably relatively to the base board 111 in the back-and-forth direction, respectively.

The four slits 112 are the slit 112A, the slit 112B, the slit 112C, and the slit 112D from right to left. The slit 112A is longer than the other slit 112B, slit 112C and slit 112D in the back-and-forth direction. Further, the slit 112B, the slit 112C and the slit 112D are formed to have the lengths equal to each other in the back-and-forth direction.

The top board 115 is a plate member attached to columns 113 extending upwardly from the base board 111. The top board 115 holds and supports the pulley 131L, 131R on the main body side, the drive pulley 132, the pulley 141 for forceps and the guide pulleys 142 between the top board and the base board 111, and rotatably supports the pulley 131L, 131R on the main body side, the drive pulley 132, the pulley 141 for forceps and the guide pulleys 142.

The shaft 23 is the cylindrical member extending forth from the main body 110 and is supported with the main body 110 rotatably around the axis L. To a base end portion on the back direction of the shaft 23, the shaft side pulley 121, which shares the axis L with the shaft 23, is provided. In the present embodiment, an explanation is given to an application of an example in which forceps (holding unit 27) are provided at a front end portion on the forth direction of the shaft 23.

The shaft side pulley 121 is a member provided at the base end portion on the back direction (on the main body 110 side) in the shaft 23 so as to be rotatably relatively to the main body 110 together with the shaft 23 and formed into a cylindrical shape. Further, on an outer peripheral face of the shaft side pulley 121, a spiral groove is formed to which a wire (funicular body) 122 for rotation is wound to control the rotation of the shaft 23.

The pair of the main body side pulleys 131L, 131R are rotatably arranged relatively to the main body 110 at positions on the base board 111 of the main body 110 between which the shaft side pulley 121 is interposed in the right-and-left direction. The main body side pulleys 131L, 131R are formed into a disk shape or a cylindrical shape, and annular grooves around which the wire 122 for rotation is wound are formed on outer peripheral faces thereof. In addition, the main body side pulley 131 is arranged on the left and the main body side pulley 131R is arranged on the right.

The wire 122 for rotation is formed into a funicular shape to transmit rotation of the main body side pulley 131R to the shaft side pulley 121 and the shaft 23. Further, the wire 122 for rotation is annularly arranged between the pair of the pulleys 131L, 131R on the shaft side, and is arranged to be wound around the outer peripheral face of the shaft side pulley 121.

The drive pulley 132 is a pulley arranged on the base board 111 of the main body 110 in the back direction of the main body side pulley 131R. The drive pulley 132 is formed into a disk shape or a cylindrical shape, and an annular groove to which a wire (other funicular body) 133 for transmission is wound is formed on an outer peripheral face thereof.

Between the drive pulley 132 and the main body side pulley 131R, a slit 112A is provided to be used for rotational drive of the shaft 23, and to the slit 112A, the movable part 151A is arranged to be used for the rotational drive of the shaft 23.

The wire 133 for transmission is formed into a funicular shape to transmit relative movement of the movable part 151A with respect to the base board 111 of the main body 110 in the back-and-forth direction to the main body side pulley 131R. Further, the wire 133 for transmission is annularly arranged between the drive pulley 132 and the main body side pulley 131R and part of the wire is fixedly attached to the movable part 151A.

The pulley 141 for forceps is a pulley arranged backward on the base board 111 of the main body 110 in the vicinity of the axis L. The pulley 141 for forceps is formed into a disk shape or a cylindrical shape, and an annular groove around which a wire 143 for forceps is wound is formed on the outer peripheral face thereof.

Between the pulley 141 for forceps and the shaft side pulley 121, the guide pulleys 142 and the slits 112B and the slit 112C are arranged. The movable part 151C is arranged to the slit 112C to drive the forceps.

The guide pulleys 142 are pulleys to lead the wire 143 for forceps from the main body 110 side to the shaft side pulley 121 and into the shaft 123. In the present embodiment, explanation is made for an application of an example in which four guide pulleys 142 are arranged between the slit 112B and the slit 112C, and the shaft side pulley 112 on the base board 111 of the main body 110. The guide pulleys 142 are formed into a disk shape or a cylindrical shape, and annular grooves to which the wire 143 for forceps is wound are formed on outer peripheral faces thereof.

The number of the guide pulleys 142 are four or may be more or less than four. Further, the relative arrangement relation among the plurality of the guide pulleys 142 is not particularly limited as long as the it is an arrangement relation in which the wire 143 for forceps is led to the shaft side pulley 121 and into shaft 23.

The wire 143 for forceps is formed into a funicular shape to transmit relative movement of the movable part 151C with respect to the base board 111 of the main body 110 in the back-and-forth direction to the forceps arranged on a forward end portion on the shaft 23. Further, the wire 143 for forceps is annularly arranged between the pulley 141 for forceps and the forceps (the holding unit 27), and part of the wire is fixedly attached to the movable part 151C.

Namely, the wire 143 for forceps corresponds to the drive wires 29 of the reference example 6, front end portions of which are fixed to the movable part 33 of the joint function part 25, passes through the flexible tube 35 and the base part 31, and passes inside the shaft 23 to be led out to the main body 110. Then, base end portions of the wire 143 for forceps are connected through the guide pulleys 142 to the pulley 141 for forceps to be configured integrally therewith. In this way, the wire 143 for forceps is annularly configured through the movable part 33 of the joint function part 25 and the pulley 141 for forceps.

It should be noted that, in the present embodiment, explanation is made for an application of an example in which the wire 143 for forceps is fixed and attached to the movable part 151C. The wire 143 for forceps may be fixed and attached to the movable part 151B or 151D and is not limited particularly. In the present embodiment, one of the movable part 151B and the movable part 151D may be connected to, for example, the wire for forceps to bend the joint function part 25 toward a different direction and the other thereof may be connected to, for example, the flexible member such as a forceps wire or a push-pull cable to open/close the holding unit 27.

The movable parts 151 are arranged to the respective four slits 112 provided on the base board 111 of the main body 110, and are arranged relatively movably with respect to the base board 111 of the main body 110 along the slits 112 in the back-and-forth direction. The movable parts 151 are moved by driving force transmitted from the master-slave type surgical robot to which the instrument 100 for a surgical assistance robot is attached.

In the four slits 112, the movable part 151A is arranged to the slit 112A, the movable part 151B is arranged to the slit 112B, the movable part 151C is arranged to the slit 112C, and the movable part 151D is arranged to the slit 112D.

Next, explanation will be made for operation in the instrument 100 for a surgical assistance robot having the aforementioned structure. First, explanation will be made for operation to rotate the shaft 23, then explanation will be made for operation to bend the joint function part 25.

When rotating the shaft 23, driving force is applied from the outside to move the movable part 151A backward or forward along the slit 112A. For example, when the movable part 151A moves forward, the part in the wire 133 for transmission attached to the movable part 151A also moves forward.

The annular wire 133 for transmission moves in a direction to rotate counterclockwise when view from the above. With this movement of the wire 133 for transmission, the main body side pulley 131R is rotated counterclockwise. The counterclockwise rotation of the main body side pulley 131R is transmitted to the wire 122 for rotation wound around the main body side pulley 131R.

The wire 122 for rotation is rotated counterclockwise, the movement of the wire 122 for rotation is transmitted to the shaft side pulley 121, and the shaft side pulley 121 is rotated around the axis L. The rotational direction of the shaft side pulley 121 is fixed based on the winding direction of the wire 122 for rotation to the shaft side pulley 121. When the movable part 151A moves backward, the movement is reversed to the above.

Next, when driving so as to bend the joint function part 25, driving force is applied from the outside to move the movable part 151C backward or forward along the slit 112C. For example, when the movable part 151C moves forward, the part in the wire 143 for forceps attached to the movable part 151C also moves forward.

The annular wire 143 for forceps moves in a direction to rotate clockwise when view from the above. This movement of the wire 143 for forceps is transmitted to the movable part 33 of the holding unit 27, and the joint function part 25 is driven so as to be bent.

According to the instrument 100 for a surgical assistance robot having the aforementioned structure provides the same effects as the reference examples 1 and 6. Additionally, the pair of the pulleys 131A, 131R on the main body side are arranged to interpose the shaft side pulley 121 arranged on the shaft 23 therebetween, and the wire 122 for rotation is wound around the shaft side pulley 121 and the pair of the main body side pulleys 131L, 131R. Transmitting the driving force to the wire 122 for rotation rotates the shaft side pulley 121 and the shaft 23.

The shaft side pulley 121 is arranged between the pair of the main body side pulleys 131L, 131R, the wire 122 for rotation extending from the one main body side pulley 131R is wound around the shaft side pulley 121, and then the wire 122 for rotation is wound around the other main body side pulley 131. Accordingly, it is easy to prevent force from acting on the shaft side pulley 121 and the shaft 123 in the orthogonal direction relative to the axial direction of the shaft 123 (in other words, a radial direction). As a result, it is easy to reduce drive load to rotate the shaft 23.

Further, it is easy to reduce the area in the shaft side pulley 121 around which the wire 122 for rotation is wound, i.e., easy to shorten the length of the shaft side pulley 121 in the axial direction.

It is easy to suppress a loss generated when converting the straight movement of the movable part 151A into the rotational movement of the shaft 123 by transmitting the movement of the movable part 151A in the straight direction to the main body side pulleys 131 through the wire 133 for transmission.

In the plurality of the movable parts 151A, 151B, 151C, 151D arranged side by side, the wire 133 for transmission is attached to the movable part 151A arranged at the end portion, thereby to easily make the instrument 100 for a surgical assistance robot compact by comparison with a case that the wire 133 for transmission is attached to the movable parts 151B, 151C arranged inside.

### EMBOIDMENT 2

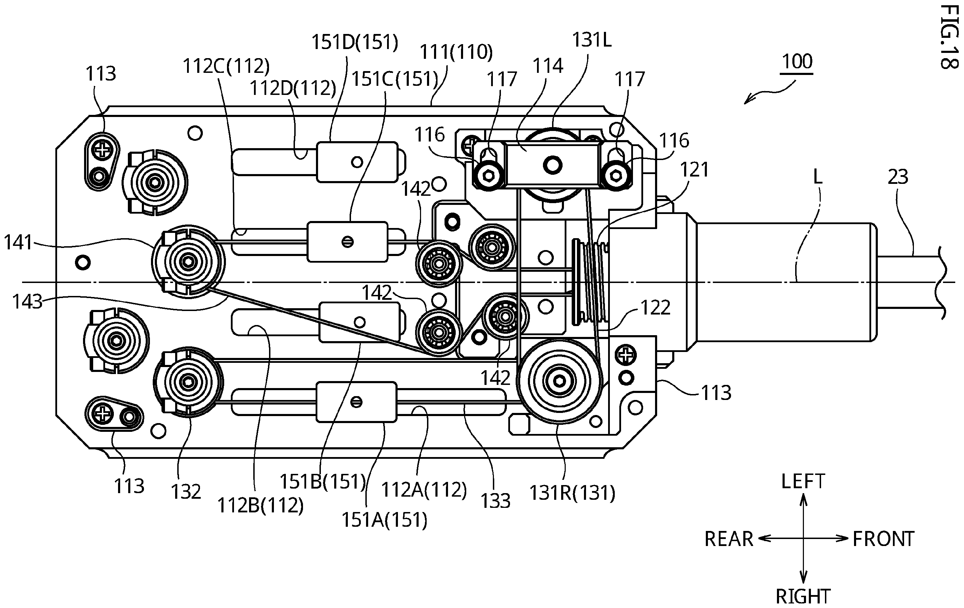

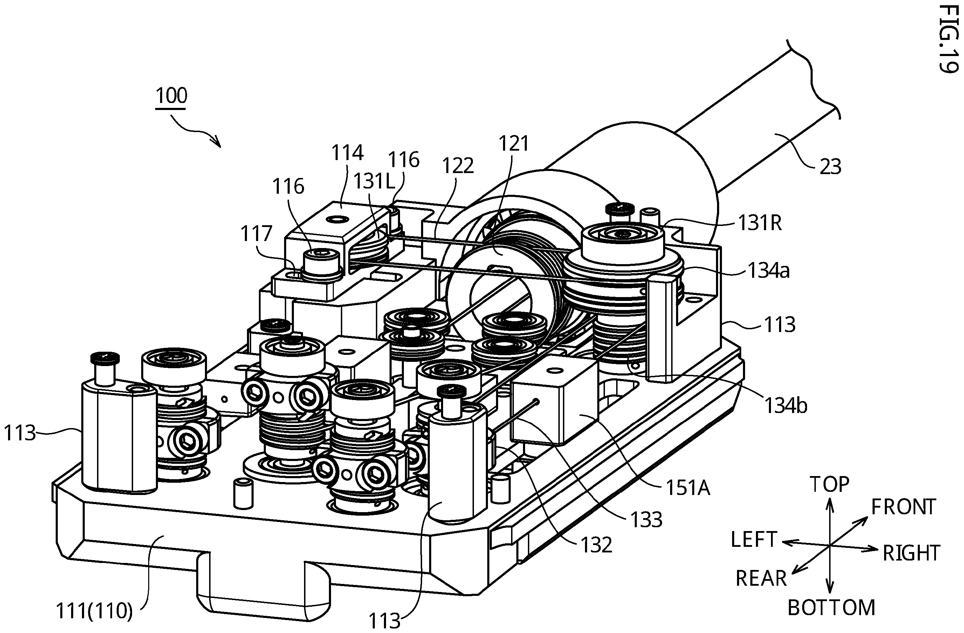

FIG. 18 is a plan view illustrating an internal structure of a main body of an instrument for a surgical assistance robot according to the embodiment 2 of the present invention, and FIG. 19 is a partial perspective view explaining the internal structure of the main body of the instrument for a surgical assistance robot of FIG. 18. Although the basic structure of an instrument 100 for a surgical assistance robot of the present embodiment is the same as of the embodiment 1, it is different from the embodiment 1 in the structure relevant to a pair of main body side pulleys. In the present embodiment, therefore, a structure relevant to the pair of the main body side pulleys will be explained using FIG. 18 and FIG. 19, and explanation for the other structure and the like will be omitted.

An adjustment part 114 is provided on a base board 111 of a main body 111 in the instrument 100 for a surgical assistance robot of the present embodiment, as illustrated in FIG. 18 and FIG. 19. The adjustment part 114 rotatably supports a main body side pulley 131, and supports the main body side pulley 131L relatively movably with respect to the base board 111 in a right-and-left direction.

In other words, it supports the main body side pulley 131 so as to approach and separate with respect to a main body side pulley 131R. On the adjustment part 114, a long hole 117 elongated in the right-and-left direction is provided. The adjustment part 114 is attached to the base board 111 by a fixing member 116 such as a screw passing through the long hole 117.

Further, the main body side pulley 131R of the instrument 100 for a surgical assistance robot of the present embodiment is provided with a large diameter pulley portion 134a with a relatively large diameter and a small diameter pulley portion 134b with a relatively small diameter. The large diameter pulley portion 134a is formed on an upward side of the main body side pulley 131R and the small diameter pulley portion 134b is formed on a downward side of the main body side pulley 131R.

On the large diameter pulley portion 134a, an annular groove around which a wire 122 for rotation is wound is formed, and on the small diameter pulley portion 134b, an annular groove around which a wire 133 for transmission is wound is formed.

In the present embodiment, the explanation has been made for the application of the example in which the diameter of the portion around which the wire 122 for rotation is wound is relatively large and the diameter of the portion around which the wire 133 for transmission is wound is relatively small in the main body side pulley 131R. The diameter of the portion around which the wire 122 for rotation is wound may be relatively small and the diameter of the portion around which the wire 133 for transmission is wound may be relatively large.

The instrument 100 for a surgical assistance robot having the aforementioned structure provides the same effect as the embodiment 1. Additionally, as illustrated in FIG. 18 and FIG. 19, the fixing member 116 is loosened and the adjustment part 114 is moved relatively to the base board 111 in the right-and-left direction, thereby to adjust the distance between the main body side pulley 131 and the main body side pulley 131R. In other words, the tension of the wire 122 for rotation is adjusted so as to become desired tension. The fixing member 116 is again tightened after the adjustment, thereby to fix the adjustment part 114 relatively to the base board 111 and fix the distance between the main body side pulley 131 and the main body side pulley 131R.

Further, when the driving force is applied from the outside to move the movable part 151A backward or forward along the slit 112A, the wire 133 for transmission performs the rotational movement. With the movement of the wire 133 for transmission, the main body side pulley 131R also performs the rotation to be transmitted to the wire 122 for transmission.

At this time, the moving amount of the wire 133 for transmission is amplified to be transmitted to the wire 122 for rotation according to ratio in diameter between the small diameter pulley portion 134b around which the wire 133 for transmission is wound and the large diameter pulley portion 134a around which the wire 122 for rotation is wound.

In addition, although the explanation has been made for the application of the example in which the moving amount of the wire 133 for transmission is amplified to be transmitted to the wire 122 for rotation in the present embodiment, the moving amount of the wire 133 for transmission may be reduced to be transmitted to the wire 122 for rotation in the present embodiment by varying the ratio between the diameter of the pulley portion around which the wire 133 for transmission is wound and the diameter of the pulley portion around which the wire 122 for rotation is wound.

According to the above structure, it is easy to adjust the tension acting on the wire 122 for rotation wound between the pair of the main body side pulleys 131L, 131R by adjusting the distance between the pair of the main body side pulleys 131L, 131R. Additionally, it is easy to suppress generation of variation in assembly of the instrument 100 for a surgical assistance robot due to ease of the adjustment of the tension. Further, it is easy to increase precision of estimation of external force.

In this way, it is easy to adjust the ratio between the moving amount of the movable part 151A in the straight direction and the rotating amount of the shaft 23 to a desired value by winding the wire 122 for rotation around the large diameter pulley portion 134a and winding the wire 133 for transmission around the small diameter pulley portion 134b.

It should be noted that the technical scope of the present invention is not limited to the above embodiments and various modifications may be made within a range not departing from the aim of the invention. For example, it is not limited to the above embodiments to which the present invention is applied, the present invention may be applied to an embodiment being a combination of those embodiments, and the application is not particularly limited.

### DESCRIPTION OF REFERENCE NUMERALS

1 Bending structure 3 Flexible member 5 Outer coiled part 5a, 7a Wire 5b, 7b Coil 5c Gap 7 Inner coiled part 25 Joint function part 27 Holding unit (End effector) 31 Base part 33 Movable part 35 Flexible tube 100 Instrument for a surgical assistance robot

## Claims

1. An instrument for a surgical assistance robot, comprising:
a main body;
a shaft being a rod-shaped member extended from the main body;
an end effector for surgical operation provided at a front end of the shaft in an axial direction; and
a joint function part provided at the front end to bend with respect to the axial direction of the shaft, thereby to enable an orientation of the end effector to be changed, wherein
the joint function part has a bending structure that enables the bending, and
the bending structure comprises:
an outer coiled part formed of a wire which is wound in a coiled shape to have a plurality of coils in the axial direction; and
an inner coiled part formed of a wire which is wound in a coiled shape to have a plurality of coils in the axial direction and arranged in the outer coiled part, wherein
the outer coiled part has a plurality of gaps to distance adjacent coils in the axial direction, and
the coils of the inner coiled part are provided so as to correspond to the gaps of the outer coiled part and fit between the adjacent coils while being in contact with the adjacent coils of the outer coiled part.

2. The instrument for a surgical assistance robot according to claim 1, wherein
the outer coiled part has the gaps in respective interspaces of the adjacent coils in the axial direction.

3. The instrument for a surgical assistance robot according to claim 1 or 2, wherein
a movable length in which the inner coiled part is movable in a diametral direction relatively to an axis of the outer coiled part is equal to or less than half of (a diameter of the outer coiled part - a diameter of the inner coiled part).

4. The instrument for a surgical assistance robot according to any one of claims 1-3, further comprising:
a restricting member to restrict movement of the inner coiled part so that the movable length is equal to or less than half of (the diameter of the outer coiled part - the diameter of the inner coiled part).

5. The instrument for a surgical assistance robot according to claim 4, wherein
the restricting member is a flexible member.

6. The instrument for a surgical assistance robot according to claim 5, wherein
the instrument passes the flexible member movably in the axial direction and is bendable as well as the flexible member.

7. The instrument for a surgical assistance robot according to any one of claims 1-6, wherein
the inner coiled part and the outer coiled part are formed separately and the inner coiled part is screwed into the outer coiled part.

8. The instrument for a surgical assistance robot according to any one of claims 1-7, wherein
the outer coiled part brings the adjacent coils into contact with each other when bending at a predetermined angle.

9. The instrument for a surgical assistance robot according to any one of claims 1-8, wherein
the joint function part comprises a base part on the shaft side and a movable part supporting the end effector and configured to displace relatively to the base part, and
the bending structure is provided between the base part and the movable part to bend according to displacement of the movable part relative to the base part.

10. The instrument for a surgical assistance robot according to claim 9, further comprising:
a flexible tube being interposed between the base part and a front end part and being extendable and compressible in the axial direction, wherein
the bending structure is arranged along an axial center portion of the flexible tube in the axial direction.

11. An instrument for a surgical assistance robot, comprising:
a main body;
a shaft being a rod-shaped member extended from the main body;
an end effector for surgical operation provided at a front end of the shaft in an axial direction; and
a joint function part provided at the front end to bend with respect to the axial direction of the shaft, thereby to enable an orientation of the end effector to be changed, wherein
the joint function part has a bending structure that enables the bending, and
the bending structure is bendable relatively to the axial direction, has a length of a central axis in the axial direction that is approximately constant before, after and during bending based on that an inner diametral side of the bending is compressed and an outer diametral side of the bending is extended when the bending is performed according to external force, and restricts compression in the axial direction when the bending is not performed.
